# FASCICULE DE BREVET EUROPEEN

(11) **EP 1 840 125 B1**
(45) Date de publication et mention de la délivrance du brevet: **24.06.2009**
(21) Numéro de dépôt: 07013954.8
(22) Date de dépôt: 27.08.2003
(51) Int. Cl.: C07C 69/00

(54) **Intermédiaires pour la production de dioxane-2-alkylcarbamates**
Zwischenprodukte für die Herstellung von Dioxan-2-alkylcarbamaten
Intermediates for the preparation of dioxane-2-alkyl carbamates

(30) Priorité: 29.08.2002 FR 0210707
(43) Date de publication de la demande: 03.10.2007
(62) Demande divisionnaire de: 03758282.2
(73) Titulaire: sanofi-aventis, 75013 Paris (FR)
(72) Inventeur: Abouabdellah, Ahmed, 75013 Paris (FR); Bas, Michèle, 34570 Pignan (FR); Dargazanli, Gihad, 94230 Cachan (FR); Hoornaert, Christian, 92160 Antony (FR); Li, Adrien Tak, 92260 Fontenay aux Roses (FR); Medaisko, Florence, 94100 Saint Maur des Fosses (FR)
(74) Mandataire: Gaslonde, Aude

(56) Documents cités:
- EP-A- 0 461 958
- WO-A-97/20836

## Description

L'invention a pour objet des composés répondant à la formule générale (III). 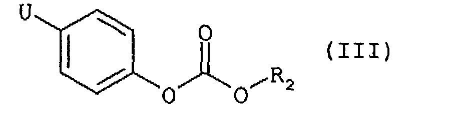 dans laquelle
R₂ représente un groupe de formule générale CHR₃CONHR₄ dans laquelle R₃ représente un atome d'hydrogène ou un groupe méthyle et R₄ représente un atome d'hydrogène ou un groupe C₁₋₃-alkyle, C₃₋₅-cycloalkyle ou (pyxidin-4-yl)méthyle ; et U représente un atome d'hydrogène ou un groupement nitro.

Les composés de formule générale (III) peuvent par exemple être utiles comme intermédiaires de synthèse des composés de formule générale (I) 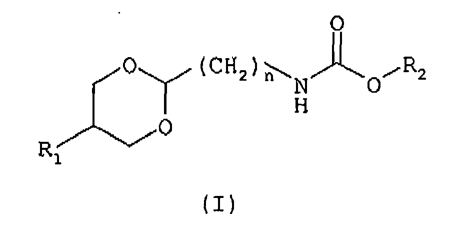 dans laquelle
R₁ représente un groupe phényle ou naphtalènyle éventuellement substitué par un ou plusieurs atomes d'halogène ou groupes hydroxy, cyano, nitro, C₁₋₃-alkyle, C₁₋₃yalcoxy, trifluorométhyle, trifluorométhoxy, benzyloxy, C₃₋₆-cycloalkyl-O- ou C₃₋₆-cycloalkylC₁₋₃-alcoxy ; R₂ représente
soit un groupe de formule générale CHR₃CONHR₄ dans laquelle R₃ représente un atome d'hydrogène ou un groupe méthyle et R₄ représente un atome d'hydrogène ou un groupe C₁₋₃-alkyle, C₃₋₅-cycloalkyle ou (pyridin-4-yl)méthyle,
soit un groupe 2,2,2-trifluoroéthyle,
soit un groupe (imidazol-2-yl)méthyle,
soit un groupe (benzimidazol-2-yl)méthyle,
soit un groupe phényle éventuellement substitué par un ou plusieurs atomes d'halogène ou groupes cyano, nitro, C₁₋₃-alkyle, C₁₋₃-alcoxy, trifluorométhyle ou trifluorométhoxy ; et
n représente un nombre allant de 1 à 3.

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères cis ou *trans*. Ces énantiomères, diastéréoisomères et stéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention. Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 6, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple, un groupe C₁₋₃-alkyle représente une chaîne carbonée de 1 à 3 atomes de carbone, linéaire ou ramifiée, plus particulièrement un méthyle, éthyle, propyle, isopropyle ;
- cycloalkyle, un groupe alkyle cyclique, par exemple, un groupe C₃₋₅-cycloalkyle représente une chaîne carbonée cyclique de 3 à 5 atomes de carbone, plus particulièrement un cyclopropyle, cyclobutyle, cyclopentyle ;
- alcoxy, un groupe alkyloxy à chaîne aliphatique saturée, linéaire ou ramifiée ;
- atome d'halogène, un fluor, un chlore, un brome ou un iode.

Ainsi une méthode de préparation des composés de formule générale (I)(schéma 1) consiste à faire réagir une amine de formule générale (II), dans laquelle R₁ et n sont tels que définis dans la formule générale (I), avec un carbonate de formule générale (III), dans laquelle U représente un atome d'hydrogène ou un groupement nitro et R₂ est tel que défini dans la formule générale (I), dans un solvant tel que le toluène ou le dichloroéthane, à une température comprise entre 0 et 80°C.

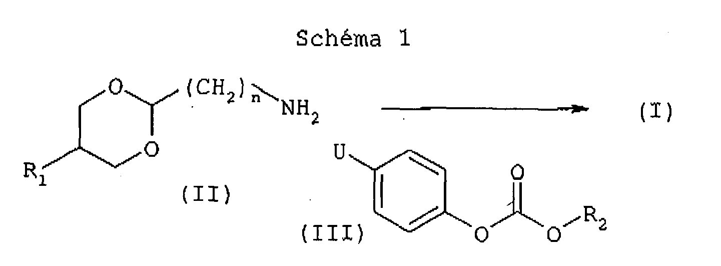

Les carbonates de formule générale (III) peuvent être préparés selon toute méthode décrite dans la littérature, par exemple par réaction d'un alcool de formule générale HOR₂ avec le chloroformiate de phényle ou de 4-nitrophényle, en présence d'une base telle que la triéthylamine ou la diisopropyléthylamine.

Les amines de formule générale (II) peuvent être préparées selon les méthodes de préparation décrites dans les demandes de brevets EP0461958, WO97/20836, WO98/55474, éventuellement adaptées suivant des techniques connues de l'homme du métier.

Les exemples qui vont suivre illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
Les numéros indiqués entre parenthèses dans les titres des exemples correspondent à ceux de la 1ère colonne du tableau ci-après.

### Exemple 1

### trans-3-[5-(6-méthoxynaphtalén-1-yl)-1,3-dioxan-2-yl]propylcarbamate de 1H-imidazol-2-ylméthyle.

### 1.1 (1-triphénylméthyl-1H-imidazol-2-yl)méthylcarbonate de phényle.

A partir de 3 g (8,80 mmoles) de 1-triphénylméthyl-1*H-*imidazole-2-méthanol (J. Het. Chem., (1995), 32, 903-906) et 1,1 ml (8,80 mmoles) de chloroformiate de phényle, on obtient 3,9 g de produit qu'on utilise tel quel dans l'étape suivante.

### 1.2 trans-3-[5-(6-méthoxynaphtalén-1-yl)-1,3-dioxan-2-yl]propylcarbamate de (1-triphénylméthyl-1H-imidazol-2-yl)méthyle.

A partir de 2,5 g (8,28 mmoles)de trans-3-[5-(6-méthoxynaphtalèn-1-yl)-1,3-dioxan-2-yl]propanamine et 3,8 g (8,28 mmoles) de (1-triphénylméthyl-1*H*-imidazol-2-yl)méthylcarbonate de phényle, obtenu à l'étape 1.1, on obtient 3,2 g d'un solide sous forme amorphe.

### 1.3 trans-3-[5-(6-méthoxynaphtalèn-1-yl)-1,3-dioxan-2-yl]propylcarbamate de 1H-imidazol-2-ylméthyle.

A une solution de 1,9 g (2,83 mmoles) de trans-3-[5-(6-méthoxynaphtalèn-1-yl)-1,3-dioxan-2-yl]propylcarbamate de (1-triphénylméthyl-1*H*-imidazol-2-yl)méthyle, obtenu à l'étape 1.2, dans 150 ml de dichlorométhane, on ajoute goutte à goutte, à température ambiante, une solution de 0,6 ml (2,83 mmoles) d'acide trifluoroacétique dans 2 ml de dichlorométhane et on agite le mélange à température ambiante pendant 12 h. On concentre sous pression réduite, on reprend le résidu avec du dichlorométhane et une solution aqueuse saturée d'hydrogénocarbonate de sodium, on sépare la phase organique, on la lave avec une solution aqueuse saturée de chlorure de sodium, on la sèche sur sulfate de sodium, on concentre sous pression réduite et on purifie le résidu par chromatographie sur gel de silice en éluant avec un mélange 98/2/0,2 de dichlorométhane, méthanol et ammoniaque.
Après recristallisation dans l'acétate d'éthyle on obtient finalement 0,820 g de produit pur.
Point de fusion : 130-132°C.
RMN ¹H : (CDCl₃) δ(ppm) 10,0 (m large, 1H) ; 8,10 (d, 1H) ; 7,65 (d, 1H); 7,35 (dd, 1H); 7,25 (d, 1H); 7,15 (dd, 1H); 7,05 (dd, 1H); 7,00 (s, 2H); 5,15 (m+s, 3H); 4,70 (t, 1H); 4,30 (m, 2H); 3,95-3,90 (m, 6H); 3,30 (m, 2H); 1,85 (m, 4H).

### Exemple 2

### trans-3-[5-(6-cyclopropylméthoxy-naphtalèn-1-yl)-1,3-dioxan-2-yl]-propylcarbamate de 2,2,2-trifluoxoéthyle.

A une suspension de 0,205 g (1,01 mmoles) de chloroformiate de 4-nitrophényle et de 0,555 g (2,02 mmoles) de *N,N-*diisopropylaminoéthylpolystyrène (Ps-DIEA, 2% DVB, titre = 3,66 mmoles/g) dans 7,1 ml de dichlorométhane, on ajoute, goutte à goutte et à température ambiante 0,075 ml (1,01 mmoles) de 2,2,2-trifluoroéthanol. On agite le mélange sous agitation orbitalaire et à température ambiante pendant 16 heures. On filtre la résine à travers une cartouche munie d'un fritté et on rince avec 4 ml de dichlorométhane. On concentre le filtrat sous pression réduite et on reprend le résidu huileux ainsi obtenu dans 3,5 ml de 1,2-dichlorométhane. Successivement, on ajoute 0,134 ml (0,78 mmoles) de *N,N*-diisopropyléthylamine et 0,205 g (0,6 mmoles) de 3-[5-(6-cyclopropylméthoxy-naphtalèn-1-yl)-1,3-dioxan-2-yl]propylamine. On chauffe ce mélange réactionnel à 60°C pendant 16 heures. Après refroidissement, on lave avec 20 ml d'une solution de soude 1N. On sépare les phases et on filtre la phase organique à travers un fritté hydrophobe. On concentre le filtrat sous pression réduite et on purifie le résidu par chromatographie sur gel de silice en éluant par un mélange 80/20 de cyclohexane et d'acétate d'éthyle. Après lavage dans 5 ml de diisopropyléther, on obtient 0,076 g de solide blanc.
LC-MS : M+H= 468
Point de fusion : 105-107°C
RMN ¹H : (CDCl₃) δ(ppm) : 8,15 (d, 1H); 7,65 (d, 1H); 7, 35 (dd, 1H); 7,25 (m, 1H); 7,15 (d, 1H); 7,10 (d, 1H); 5,15 (m large, 1H); 4,75 (t, 1H); 4,50 (q, 2H); 4,30 (dd, 2H); 4,10-3,80 (m, 5H); 3,40-3,20 (m, 2H); 1,90-1,65 (m, 4H); 1,45-1,20 (m, 1H); 0,75-0,60 (m, 2H); 0,50-0,35 (2H).

## Revendications

1. Composé répondant à la formule générale (III) 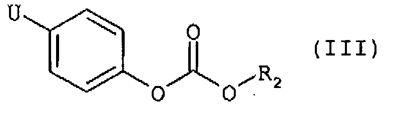 dans laquelle
R₂ représente un groupe de formule générale CHR₃CONHR₄ dans laquelle R₃ représente un atome d'hydrogène ou un groupe méthyle et R₄ représente un atome d'hydrogène ou un groupe C₁₋₃-alkyle, C₃₋₅-cycloalkyle ou (pyridin-4-yl)méthyle ; et
U représente un atome d'hydrogène ou un groupement nitro.

## Claims

1. Compound corresponding to general formula (III) 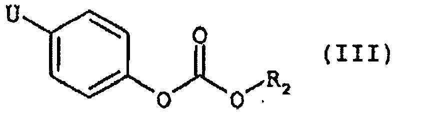 in which:
R₂ is a group of general formula CHR₃CONHR₄ in which R₃ is a hydrogen atom or a methyl group and R₄ is a hydrogen atom or a C₁₋₃-alkyl, C₃₋₅-cycloalkyl or (pyridin-4-yl)methyl group; and U is a hydrogen atom or a nitro group.

## Patentansprüche

1. Verbindung der allgemeinen Formel (III) 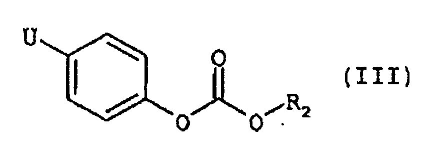 worin
R₂ für eine Gruppe der allgemeinen Formel CHR₃CONHR₄, worin R₃ ein Wasserstoffatom oder eine Methylgruppe bedeutet und R₄ ein Wasserstoffatom oder eine C₁₋₃-Alkyl-, C₃₋₅-Cycloalkyl- oder (Pyridin-4-yl)methylgruppe bedeutet, steht und U für ein Wasserstoffatom oder eine Nitrogruppe steht.
